(19) Europäisches Patentamt / European Patent Office / Office européen des brevets

(11) EP 2 067 477 B1

(12) **EUROPEAN PATENT SPECIFICATION**

(45) Date of publication and mention of the grant of the patent:
**16.04.2014 Bulletin 2014/16**

(21) Application number: **07785267.1**

(22) Date of filing: **06.08.2007**

(51) Int Cl.:
***A61K 31/704*** (2006.01) ***A61P 19/02*** (2006.01)

(86) International application number:
**PCT/CN2007/002354**

(87) International publication number:
**WO 2008/034328 (27.03.2008 Gazette 2008/13)**

(54) **NEW USE OF GINSENOSIDE COMPOUND-K FOR THE PREVENTION OF RHEUMATOID ARTHRITIS**

NEUE VERWENDUNG VON GINSENOSID-VERBINDUNG-K FÜR DIE PRÄVENTION VON RHEUMATOIDE ARTHRITIS

NOUVELLE UTILISATION DU COMPOSÉ-K GINSENOSIDE POUR PRÉVENTION DE LA POLYARTHRITE RHUMATOÏDE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **19.09.2006 CN 200610116197**

(43) Date of publication of application:
**10.06.2009 Bulletin 2009/24**

(73) Proprietors:
- **Zhejiang Hisun Pharmaceutical Co. Ltd.**
  **Zhejiang 318000 (CN)**
- **Fudan University**
  **Shanghai 200433 (CN)**
- **Shanghai Institute of Pharmaceutical Industry**
  **Shanghai 200040 (CN)**

(72) Inventors:
- **LIU, Quanhai**
  **Shanghai 200040 (CN)**
- **ZHOU, Pei**
  **Shanghai 200433 (CN)**
- **BAI, Hua**
  **Zhejiang 318000 (CN)**
- **ZHOU, Wei**
  **Shanghai 200433 (CN)**
- **LI, Jingjing**
  **Shanghai 200040 (CN)**
- **FENG, Meiqing**
  **Shanghai 200433 (CN)**
- **HUA, Moli**
  **Shanghai 200040 (CN)**
- **XU, Jingyue**
  **Shanghai 200040 (CN)**

(74) Representative: **Hannke, Christian**
**Hannke Bittner & Partner**
**Patent- und Rechtsanwälte**
**Prüfeninger Strasse 1**
**93049 Regensburg (DE)**

(56) References cited:
**WO-A1-2004/058796 CN-A- 1 717 414**
**KR-A- 20060 067 900**

- **DATABASE WPI Week 200711, Derwent Publications Ltd., London, GB; AN 2007-107826, XP008105470 & KR 2006 0 067 900 A (SONG Y W) 20 June 2006**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 067 477 B1

## Description

Technical Field

The present invention relates to a new use of ginsenoside Compound-K in manufacturing medicaments, in particular use in the manufacturing medicaments for prevention of rheumatoid arthritis.

Background of the Invention

Chinese patent (CN1570733A) disclosed the structure, the manufacturing method and the use for anti-tumor of ginsenoside Compound-K, which structural formula is as follow:

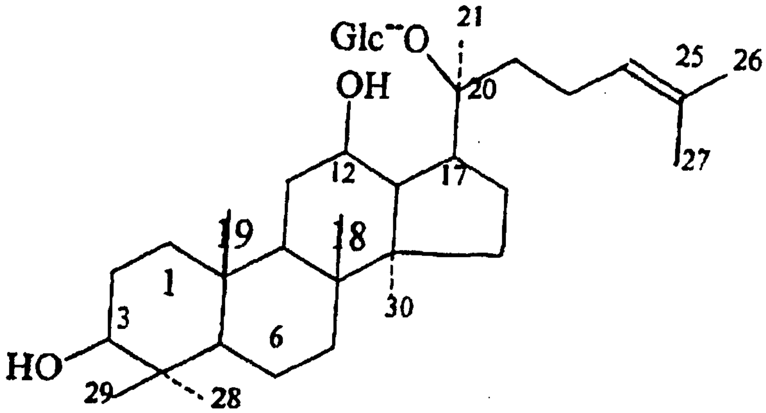

But there is no any report on the use of ginsenoside Compound-K for prevention of arthritis.
WO 2004/058 796 A1 discloses Ginsenoside Compound-K as promotor for the expression of hyaluronic acid synthetase. Since hyaluronic acid is assumed to be effective, also treatment of osteoarthritis is the subject matter of this document.
KR 2006/006 790 0 A1 discloses Ginsenoside Compound-K as active ingredient of a pharmaceutical composition for the treatment of arthritis. It discloses in vitro experiments of the effect on enzymes (MMP-13 and MMP-3) which are involved in arthritis.

Summary of the Invention

The object of the present invention is to study the possibility of using of ginsenoside Compound-K in other fields, and provides new use of ginsenoside Compound-K in manufacturing medicaments.
The present invention provides use of ginsenoside Compound-K in the manufacturing medicaments for prevention of rheumatoid arthritis.

Detailed Description of the Invention

Example 1 Primary Toxicity Test

Mice were treated by intravenous injection of ginsenoside Compound-K at the dose of 360mg/kg, their act was normal and no behavior out of the way was observed.

Example 2 Effect of ginsenoside Compound-K on carrageenan-induced paw edema in mice

1. The object of experiment: to observethe effect of the example to be tested on carrageenan-induced acute inflammation in mice

2. Materials and Reagents

1) Medicine: Ginsenoside Compound-K, at the dose of 10mg/kg, 5mg/kg, 2.5mg/kg. Indomethacin, at the dose of 10mg/kg.
Carrageenan, concentration 1% made of double distilled water.
2) Animals:

60 Kunming mice.
Body weight: 19-21 g.
Sex: male.
Number of animals in each group: 10.
Temperature in laboratory: 24~26°C; Relative humidity: 60~70%

3. Methods of experiment:

Animals were firstly divided into a large dose group: 10mg/kg, iv, a medium dose group: 5mg/kg, iv, a small dose group: 2.5mg/kg, iv, a Indomethacin group: 10mg/kg, po and a blank control group: physiological saline 10mg/kg, iv. And then the mice were administered by aforesaid method for 3 days. 1 hour after the last administration, 0.1 ml 1% carrageenan was injected into the plantar surface of the right hind paw in order to induce inflammation. The volume of the right hind paw of the mice was measured by using a hydroplethismometer before inflammation induced and every other one hour thereafter respectively. The difference of the volume of the right hind paw between before inflammation inducement and at the different time points after inflammation inducement was the swell value. Swell rate and inhibition rate were calculated. The differences among groups were compared with t test.

$$\text{Swell rate } \% = \frac{E_n - E_0}{E_0} \times 100\%$$

En = swell value at the different time points after inflammation inducement
Eo = swell value before inflammation inducement

$$\text{Inhibition rate } \% = \frac{c - t}{c} \times 100\%$$

C = swell rate of control group
T = swell rate of treatment group

4. Results

The results were shown in table 1. Ginsenoside Compound-K was able to inhibit carrageenan-induced acute inflammation in mice obviously. The most effectivel inhibition was observed in the large dose group on the 4th hour with inhibition rate 65.66% for the acute inflammation in mice. The inhibition effect of Indomethacin group amounts to the same as the small dose group.

Table 1. Effect of Ginsenoside Compound-K on Carrageenan-induced Acute Inflammation in Mice (%, n = 10, x±s)

| The swell vale at different time points after inflammation-induced (ml) | | | | | |
|---|---|---|---|---|---|
| | 0h | 1h | 2h | 4h | 6h |
| Inflammation-induced control | 1.52 ± 0.11 | 2.56 ± 0.30<br>**(68.76 ± 18.85)** | 2.62 ± 0.18<br>**(72.65 ± 8.90)** | 2.74 ± 0.22<br>**(80.47 ± 10.57)** | 2.62 ± 0.18<br>**(73.23 ± 16.83)** |
| High dose 10mg/kg | 1.56 ± 0.17 | 2.18 ± 0.18<br>**(40.81 ± 10.14)****<br>*<40.64>* | 2.19 ± 0.20<br>**(41.56 ± 13.38)****<br>*<42.79>* | 1.98 ± 0.20<br>**(27.64 ± 8.46)****<br>*<65.66>* | 1.91 ± 0.22<br>**(23.58 ± 15.23)****<br>*<67.80>* |

(continued)

| The swell vale at different time points after inflammation-induced (ml) | | | | | |
|---|---|---|---|---|---|
| | 0h | 1h | 2h | 4h | 6h |
| Medium dose 5mg/kg | 1.47 ± 0.13 | 2.36 ± 0.23 **(60.97 ± 18.09)** *<11.33>* | 2.35 ± 0.16 **(60.97 ± 18.41)** *<16.08>* | 2.12 ± 0.22 **(45.04 ± 18.30)**** *<44.03>* | 2.19 ± 0.19 **(49.81 ± 17.26)**** *<31.98>* |
| Low dose 2.5gmg/kg | 1.49 ± 0.15 | 2.36 ± 0.22 **(66.63 ± 7.82)** *<14.70>* | 2.43 ± 0.23 **(64.26 ± 14.54)** *<12.30>* | 2.33 ± 0.17 **(61.75 ± 11.27)**** *<29.40>* | 2.22 ± 0.15 **(53.48 ± 11.12)**** *<31.64>* |
| Indomethacin 1mg/kg | 1.47 ± 0.14 | 2.44 ± 0.19 **(67.11 ± 17.30)** *<2.39>* | 2.35 ± 0.20 **(60.95 ± 18.36)** *<16.11>* | 2.21 ± 0.11 **(51.04 ± 14.09)**** *<36.57>* | 2.26 ± 0.11 **(55.36 ± 19.90)*** *<24.41>* |
| ***P<0.05, **P<0.01 compared with inflammation-induced control group ( ) is swell rate (%)** < > *is inhibition rate (%)* | | | | | |

Example 3 Effect of ginsenoside Compound-K on carrageenan-induced paw edema in rats

1. The object of experiment: to observe the effect of example to be tested on carrageenan-induced acute inflammation in rats

2. Materials and Reagents

1) Medicine: Ginsenoside Compound-K, at the dose of 10mg/kg, 5mg/kg, 2.5mg/kg. Indomethacin, at the dose of 1mg/kg.
Carrageenan, concentration 1% made of double distilled water
2) Animals:

Rat, SD strain.
Body weight: 130~150 g.
Sex: male.
Number of animals in each group: 8.
Temperature in laboratory: 24~26°C; Relatively humidity: 60~70%

3. Methods of experiment:

Animals were firstly divided into large dose group: 10mg/kg, iv, medium dose group: 5mg/kg, iv, small dose group: 2.5mg/kg, iv, an Indomethacin group: 1mg/kg, po and a blank control group: physiological saline 10mg/kg, iv. The animals were administered by the foregoing method for 3 days. The volume of the right hind paw of the rats was measured using hydroplethismometer before inflammation induced. 1 hour after the last administration, 0.1 ml 1% carrageenan was injected into the plantar surface of the right hind paw in order to induce inflammation. The volume of the right hind paw of the rats was measured by hydroplethismometer every other hour thereafter respectively. Swell rate and inhibition rate were calculated by the same method as above. Differences among groups were compared with t test.

4. Results

The results were shown in table 2. Ginsenoside Compound-K was able to inhibit carrageenan-induced acute

inflammation in rats obviously. The most effective inhibition was observed in the large dose group, where the highest inhibition rate may reach 78.23%. The inhibition effect of Indomethacin group was in between the large dose group and the low dose group.

**Table 2. Effect of Ginsenoside Compound-K on Carrageenan-induced Acute Inflammation in Rats (%, n = 10, x±s)**

| | The swell vale at different time points after inflammation-induced (ml) | | | | | |
|---|---|---|---|---|---|---|
| | 0h | 1h | 2h | 3h | 4h | 5h |
| **inflammation-inducedcontrol** | 1.40 ± 0.10 | 1.73 ± 0.18 **(23.51 ± 10.11)** | 1.85 ± 0.15 **(32.24 ± 15.29)** | 1.98 ± 0.16 **(41.70 ± 14.21)** | 1.94 ± 0.18 **(38.84 ± 15.35)** | 1.91 ± 0.21 **(37.07 ± 17.47)** |
| **Large dose 10mg/kg** | 1.42 ± 0.10 | 1.51 ± 0.10 **(6.38 ± 7.44)**** *<72.87>* | 1.62 ± 0.11 **(13.87 ± 8.55)"** *<56.96>* | 1.65 ± 0.16 **(16.06 ± 12.41)**** *<61.49>* | 1.58 ± 0.14 **(11.51 ± 9.76)**** *<70.38>* | 1.53 ± 0.12 (8.07 ± **10.88)**** *<78.23>* |
| **Mediumdose 5mg/kg** | 1.39 ± 0.12 | 1.55 ± 0.11 **(11.87 ± 6.23)**** *<49.51>* | 1.64 ± 0.09 **(18.56 ± 9.77)*** *<42.44>* | 1.77 ± 0.12 **(28.09 ± 11.34)**** *<32.62>* | 1.66 ± 0.13 **(20.11 ± 11.20)**** *<48.22>* | 1.62 ± 0.11 **(17.13 10.13)**** *<53.80>* |
| **Low dose 2.5gmg/kg** | 1.33 ± 0.08 | 1.59 ± 0.08 **(20.22 ± 9.99)** *<13.97>* | 1.71 ± 0.13 **(29.40 ± 12.71)** *<8.80>* | 1.79 ± 0.14 **(34.80 ± 14.01)** *<16.54>* | 1.71 ± 0.12 **(29.49 ± 13.86)** *<24.08>* | 1.74 ± 0.12 **(31.22 ±** 14.12) *<15.78>* |
| **Indomethacin 1mg/kg** | 1.34 ± 0.15 | 1.68 ± 0.10 **(22.51 ± 10.19)** *<4.23>* | 1.72 ± 0.16 **(25.65 ± 14.10)** *<20.44>* | 1.82 ± 0.17 **(33.28 ± 13.54)** *<20.17>* | 1.77 ± 0.22 **(28.45 ± 15.71)** *<26.76>* | 1.69 ± 0.15 **(23.04 ± 11.45)** *<37.84>* |
| ***P<0.05, **P<0.01 compared with inflammation-induced control group ( ) is swell rate** (%) < > is *inhibition rate (%)* | | | | | | |

Example 4 Effect of ginsenoside Compound-K on adjuvant induced Adjuvant Arthritis in rats

(A) Preventive effect of ginsenoside Compound-K on adjuvant induced Adjuvant Arthritis in rats

1. Materials and Reagents

1) Medicine: Ginsenoside Compound K, at the dose of 10mg/kg, 5mg/kg, 2.5mg/kg.
Indomethacin, at the dose of 1mg/kg.

Tripterygium Wilfordii Hook, at the dose of 1.5mg/kg.
Freund's Adjuvant Complete, purchased from Sigma Company, was injected into the plantar surface of the right hind paw by 0.1ml in order to induce inflammationy in rats.
2) Animals:

Rat, SD strain.
Body weight: 130~150 g.
Sex: male.

Number of animals in each group: 8.
Temperature in laboratory: 24-26°C; Relatively humidity: 60~70%.

2. Methods of experiment:

Animals were firstly divided into large dose group: 10mg/kg, iv, medium dose group: 5mg/kg, iv, small dose group: 2.5mg/kg, iv, Indomethacin group: 1mg/kg, po, Tripterygium Wilfordii Hook group: 1.5mg/kg, po and blank control group: physiological saline 10mg/kg, iv. The volume of the right hind paw of the rats was measured by using hydroplethismometer before inflammationinduced. Then 0.1 ml Freund's Adjuvant Complete was injected into the plantar surface of the right hind paw in order to induce inflammation in rats. The rats were grouped by the foregoing division way in the succeeding day, and to be administered for 15 days. The volume of paw was measured in regular intervals. Swell rate and inhibition rate were calculated by the same method as the foregoing. Differences among groups were compared by t test.

3. Results

The results were shown in table 3. Ginsenoside Compound-K was able to prevent Adjuvant Arthritis in rats. The effect of the large dose group was slightly higher than Tripterygium Wilfordii Hook group and amounts to the same as Indomethacin, group.

**Table 3. Preventive Effect of Ginsenoside Compound-K on Carrageenan-induced Adjuvant Arthritis in Rats** (%, **n=10**, **x**±**s**)

| | The swell vale at different day after inflammation inducement (ml) | | | | | | | |
|---|---|---|---|---|---|---|---|---|
| Blank control | 0day | 2day | 4day | 6day | 9day | 11day | 13day | 15day |
| Blank control | 1.22 ± 0.09 | 1.28 ± 0.07 | 1.29 ± 0.04 | 1.29 ± 0.07 | 1.29 ± 0.06 | 1.27 ± 0.05 | 1.28 ± 0.04 | 1.29 ± 0.03 |
| Inflammation-induced control | 1.23 ± 0.10 | 2.33 ± 0.31 **(72.36 ± 14.49)** | 2.33 ± 0.31 **(89.52 ± 22.12)** | 2.33 ± 0.36 **(89.34 ± 21.13)** | 2.13 ± 0.38 **(72.42 ± 18.87)** | 2.18 ± 0.31 **(77.92 ± 25.66)** | 2.25 ± 0.27 **(82.72 ± 14.20)** | 2.22 ± 0.24 **(80.63 ± 16.37)** |
| Large dose 10mg/kg | 1.29 ± 0.08 | 2.17 ± 0.14 **(51.03 ± 8.03)**** *<29.48>* | 2.17 ± 0.14 **(68.94 ± 10.49)*** *<22.99 >* | 2.21 ± 0.15 **(71.90 ± 9.64** *<19.52>* | 2.03 ± 0.15 **(58.23 ± 11.44)** *<19.59>* | 1.98 ± 0.14 **(54.48 ± 12.39)*** *<30.08>* | 2.03 ± 0.13 **(58.04 ± 10.97)**** *<29.83>* | 1.99 ± 0.18 **(55.42 ± 14.65)**** *<31.26 >* |
| Medium dose 5mg/kg | 1.25 ± 0.06 | 2.10 ± 0.21 **(46.63 ± 11.66)**** *<35.56 >* | 2.10 ± 0.21 **(68.35 ± 15.68)*** *<23.65 >* | 2.16 ± 0.18 **(73.31 ± 17.73)** *<17.94>* | 1.97 ± 0.21 **(57.60 ± 13.83)** *<20.45>* | 1.95 ± 0.18 **(55.81 ± 11.54)*** *<28.37>* | 2.00 ± 0.18 **(59.74 ± 11,82)**** *<27.79>* | 1.96 ± 0.21 **(57.08 ± 14.00)**** *<29.21 >* |
| Low dose 2.5gmg/kg Tripterygium | 1.24 ± 0.08 | 2.14 ± 0.15 **(56.65 ± 17.83)** *<21.71>* | 2.14 ± 0.15 **(73.11 ± 15.82)** *<18.33 >* | 2.11 ± 0.10 **(70.87 ± 14.91)** *<20.67>* | 1.95 ± 0.17 **(57.25 ± 14.42)** *<20.95 >* | 2.00 ± 0.26 **(61.69 ± 21.61)** <20.82 > | 2.00 ± 0.15 **(61.24 ± 13.26)**** *<25.97 >* | 1.96 ± 0.20 **(58.02 ± 15.92)**** *<28.05 >* |

(continued)

| | The swell vale at different day after inflammation inducement (ml) | | | | | | | |
|---|---|---|---|---|---|---|---|---|
| Blank control | 0day | 2day | 4day | 6day | 9day | 11day | 13day | 15day |
| Tripterygium Wilfordii Hook 1.5mg/kg | 1.24 ± 0.05 | 1.90 ± 0.07 **(53.58 ± 3.97)** *<25.95>* | 2.24 ± 0.18 **(80.76 ± 9.19)** *<9.78>* | 2.30 ± 0.62 **(85.26 ± 46.85)** *<4.57>* | 2.12 ± 0.46 **(70.92 ± 33.95)** *<2.06 >* | 1.98 ± 0.35 **(59.06 ± 25.35)** *<24.20>* | 2.03 ± 0.25 **(63.51 ± 17.33)** *<23.22 >* | 2.01 ± 0.20 **(61.57 ± 12.50)*** <23.64> |
| Indomethacin 1mg/kg | 1.23 ± 0.11 | 1.85 ± 0.09 **(52.34 ± 13.07)** *<27.67>* | 2.08 ± 0.13 **(70.99 ± 17.35)** *<20.69>* | 2.17 ± 0.17 **(78.32 ± 23.41 )** *<12.34>* | 2.06 ± 0.23 **(69.44 ± 27.12)** *<4.11>* | 1.86 ± 0.13 **(52.68 ± 12.02)'** *<32.39 >* | 1.86 ± 0.13 **(53.00 ± 15.5)**** *<35.93>* | 1.86 ± 0.13 **(52.00 ± 11.27)**** *<35.50>* |
| ***P<0.05, **P<0,01 compared with inflammation-induced control group ( ) is swell rate** (%) < > is *is inhibition rate (%)* | | | | | | | | |

(B) Treatment effect of Ginsenoside Compound-K on adjuvant induced Adjuvant Arthritis in rats (not claimed)

1. Materials and Reagents

1) Medicine: Ginsenoside Compound-K, at the dose of 10mg/kg, 5mg/kg, 2.5mg/kg.
Indomethacin, at the dose of 1mg/kg.
Tripterygium Wilfordii Hook, at the dose of 1.5mg/kg.
Freund's Adjuvant Complete, purchased from Sigma Company, was injected into the plantar surface of the right hind paw by 0.1ml in order to induce inflammation in rats.
2) Animals:

Rat, SD strain.
Body weight: 130~150 g.
Sex: male.
Number of animals in each group: 8.
Temperature in laboratory: 24~26°C; Relative humidity: 60~70%

2. Methods of experiment:

Animals were firstly divided into large dose group: 10mg/kg, iv, medium dose group: 5mg/kg, iv, small dose group: 2.5mg/kg, iv, Indomethacin group: 1mg/kg, po, Tripterygium Wilfordii Hook group: 1.5mg/kg, po. and blank control group: physiological saline 10mg/kg, iv. The volume of the right hind paw of the rats was measured by using hydroplethismometer before inflammation induced. Then 0.1 ml Freund's Adjuvant Complete was injected into the plantar surface of the right hind paw in order to induce inflammation in rats. 20 days after inflammation induced, the rats were grouped by the foregoing division method and to be administered for 8 days. The volume of paw was measured in regular intervals. Swell rate and inhibition rate were calculated with the same method as the foregoing. Differences among groups were compared with t test.

3. Results

The results were shown in table 4. Ginsenoside Compound-K was able to cure Adjutant Arthritis in rats. The strongest treatment effect was observed in the large dose group, the best effect shown on the 26$^{th}$ day, inhibition rate may arrive at 36.15%, which was obviously higher than Tripterygium Wilfordii Hook group and Indomethacin group respectively.

Table 4. Treatment Effect of Ginsenoside Compound-K on Adjuvant Arthritis in Rats (% n = 10, x±s)

| The swell vale at different day after inflammation induced (ml) | | | | | | |
|---|---|---|---|---|---|---|
| | 0day | 19day | 21day | 23day | 26day | 30day |
| Blank control | 1.16 ± 0.06 | 1.25 ± 0.08 | 1.27 ± 0.07 | 1.28 ± 0.08 | 1.31 ± 0.06 | 1.32 ± 0.07 |
| Inflammation-induced control | 1.11 ± 0.07 | 2.19 ± 0.25<br>**(98.38 ± 19.60)** | 2.28 ± 0.22<br>**(106.21 ± 17.63)** | 2.28 ± 0.23<br>**(106.50 ± 15.80)** | 2.34 ± 0.28<br>**(112.23 ± 21.37)** | 2.27 ± 0.28<br>**(105.15 ± 18.04)** |
| Large dose 10mg/kg | 1.05 ± 0.09 | 1.97 ± 0.24<br>**(88.85 ± 23.27)**<br>*<9.69>* | 1.81 ± 0.33<br>**(72.69 ± 29.97)***<br>*<31.57>* | 1.80 ± 0.21<br>**(72.50 ± 22.18)****<br>*<31.92>* | 1.79 ± 0.24<br>**(71.66 ± 24.46)****<br>*<36.15>* | 1.80 ± 0.26<br>**(71.17 ± 17.97)****<br>*<32.32>* |
| Medium dose 5mg/kg | 1.10 ± 0.09 | 2.00 ± 0.12<br>**(81.36 ± 13.10)**<br>*<17.31>* | 2.03 ± 0.20<br>**(84.07 ± 18.91)***<br>*<20.85>* | 2.01 ± 0.23<br>**(81.81 ± 14.58)****<br>*<23.19>* | 2.00 ± 0.19<br>**(81.48 ± 11.30)****<br>*<27.40>* | 2.03 ± 0.25<br>**(83.77 ± 17.65)***<br>*<20.33>* |
| Low dose 2.5gmg/kg | 1.06 ± 0.09 | 1.96 ± 0.16<br>**(84.26 ± 14.31)**<br>*<14.36>* | 2.00 ± 0.07<br>**(89.42 ± 18.47)**<br>*<15.81>* | 1.91 ± 0.08<br>**(80.92 ± 16.84)****<br>*<24.02>* | 1.92 ± 0.08<br>**(81.13 ± 10.03)****<br>*<27.71>* | 1.96 ± 0.16<br>**(84.80 ± 19.66)***<br>*<19.36>* |
| Tripterygium Wilfordii Hook 1.5mg/kg | 1.16 ± 0.05 | 2.03 ± 0.14<br>**(75.70 ± 13.13)***<br>*<23.06>* | 1.98 ± 0.08<br>**(71.88 ± 11.09)****<br>*<32.33>* | 2.06 ± 0.16<br>**(79.07 ± 16.20)****<br>*<25.75>* | 2.08 ± 0.11<br>**(80.82 ± 13.T6)****<br>*<27.99 >* | 2.07 ± 0.16<br>(79.28 ± **14.25)****<br>*<24.61>* |
| Indomethacin 1mg/kg | 1.14 ± 0.05 | 2.04 ± 0.20<br>**(80.03 ± 17.69)**<br>*<18.65>* | 2.11 ± 0.17<br>**(85.98 ± 15.49)***<br>*<19.05>* | 2.11 ± 0.17<br>**(86.16 ± 15.90)***<br>*<19.09>* | 2.15 ± 0.16<br>**(89.32 ± 16.25)***<br>*<20.41>* | 2.18 ± 0.23<br>**(92.39 ±** 20.26)<br>*<12.14>* |
| ***P<0.05 , **P<0.01 compared with inflammation-induced control group ( ) is swell rate (%)** < > is *inhibition rate (%)* | | | | | | |

Example 5 Effect of ginsenoside Compound-K on Collagen Type II (CII) induced arthritis in rats

1. Materials and Reagents

1) Medicine: Ginsenoside Compound-K, at the dose of 10mg/kg, 5mg/kg, 2.5mg/kg.
Enbrel: at the dose of 9mg/kg by subcutaneous injection every 4 days.
Tripterygium Wilfordii Hook, at the dose of 1.5mg/kg.
Inflammation-induced agent: Collagen Type II was dissolved in 0.1mol/L acetic acid to make final concentration be 2mg/ml collagen solution, and held in 4°C overnight. The Collagen Type II solution was added to cold Freund's Adjuvant Incomplete by drops (Collagen Type II: Freund's Adjuvant Incomplete=1:1) on the next day, emulsified completely (Type II Collagen solution: Freund's Adjuvant Incomplete =1:1), and the final concentration of Collagen Type II was lmg/ml.. The Collagen Type II emulsion was reserved in a refrigerator at 4°C.
Immune method: Each rat was immunized by being injected subcutaneously at 5 points on the back with 0.1ml

emulsion in each point, total of 5ml (contained 0.5mg Collagen Type II), and immunized again in the same way 7 days later. An emulsion of 0.1mol/L acetic acid and Freund's Adjuvant Incomplete was injected in control group. The swell values of the arthrosis in right hind ankle and foot were measured respectively by using a hydroplethismometer before injection and 20 days after immunization.

2) Animals:

Rat, SD strain.
Body weight: 140~160 g.
Sex: male.
Number of animals in each group: 8.
Temperature in laboratory: 24~26°C, Relatively humidity: 60~70%

2. Preventive effect of ginsenoside Compound-K on Collagen Type II (CII) induced arthritis in rats

2.1 Methods of experiment:

The Collagen Type II solution, which had been dissolved in acetic acid and held overnight, was adjusted to concentration 2mg/ml, then added by drops slowly to Freund's Adjuvant Incomplete in proportion of 1:1 . After the mixture was emulsified completely, each rat was injected subcutaneously at 5 points on the back with 0.1ml emulsion in each point (total 0.5ml in each rat, containing 0.5mg Collagen Type II) for the first immunization. 7 days later the second immunization was repeated in the same way. The animals of control group were treated in a similar way, but the immune components did not contain collagen.

The animals were divided into large dose group: Ginsenoside Compound-K, 10mg/kg, iv, medium dose group: Ginsenoside Compound-K, 5mg/kg, iv, small dose group: Ginsenoside Compound-K, 2.5mg/kg, iv, Tripterygium Wilfordii Hook group: 1.5mg/kg, po-, Enbrel group: 9mg/kg, sc and blank control group: physiological saline 10mg/kg, iv. The animals of administration groups were administered at the first immunization and stopped administering 8 days after the first immunization. The swell value of arthrosis in ankle and foot was measured by using hydroplethismometer before immunization and on day 20, 22, 24, 27 and 29 after the first immunization respectively. Swell rate and inhibition rate were calculated by the same method as the foregoing. Differences among groups were compared with t test. When the treatment finished, the arthroses in ankle and foot were taken for pathological examination.

2.2 Results

The results were shown in table 5. Ginsenoside Compound-K were able to prevent Collagen Type II induced arthritis. The strongest effect was observed in the large dose group, which was higher obviously than the Tripterygium Wilfordii Hook group and amounts to the Enbrel group.

**Table 5. Preventive Effect of Ginsenoside Compound-K on Collagen Type II (CII) Induced Arthritis in Rats (%, n = 8, x±s)**

| The swellvale at different daysafter inflammationinduced (ml) | | | | | | |
|---|---|---|---|---|---|---|
| | 0day | 20day | 22day | 24day | 27day | 29day |
| Blank control | 1.09 ± 0.05 | 1.10 ± 0.05 | 1.12 ± 0.02 | 1.10 ± 0.06 | 1.10 ± 0.06 | 1.10 ± 0.06 |
| Inflammation-induced control | 1.12 ± 0.05 | 1.59 ± 0.33 **(52.50 ± 32.54)** | 1.58 ± 0.31 **(51.56 ± 29.84)** | 1.59 ± 0.32 **(52.53 ± 31.73)** | 1.59 ± 0.26 **(50.64 ± 25.40)** | 1.48 ± 0.30 **(43.85 ± 26.39)** |
| Large dose 10mg/kg | 1.08 ± 0.07 | 1.37 ± 0.26 **(35.19 ± 19.75)** *<32.97 >* | 1.30 ± 0.23 **(27.06 ± 19.03)** *<47.51 >* | 1.31 ± 0.21 **(27.16 ± 16.39)** *<48.29 >* | 1.32 ± 0.19 **(28.05 ± 14.52)** *<44.61 >* | 1.32 ± 0.23 **(28.86 ± 19.06)** *<34.19>* |

(continued)

| The swellvale at different daysafter inflammationinduced (ml) | | | | | | |
|---|---|---|---|---|---|---|
| | 0day | 20day | 22day | 24day | 27day | 29day |
| Medium dose 5mg/kg | 1.11 ± 0.06 | 1.43 ± 0.23<br>**(37.27 + 15.30)**<br>*<29.01 >* | 1.41 ± 0.22<br>**(35.00** ± **15.12)**<br>*<32.11 >* | 1.45 ± 0.17<br>**(35.95** ± **11.21)**<br>*<31.58>* | 1.41 ± 0.23<br>**(34.66** ± **13.01)**<br>*<31.57>* | 1.39 ± 0.18<br>**(31.12** ± **13.81)**<br>*<29.04>* |
| Low dose 2.gmg/kg | 1.09 ± 0.04 | 1.39 ± 0.24<br>**(40.29** ± **18.03)**<br>*<23.26 >* | 1.38 ± 0.24<br>**(38.98** ± **18.96)**<br>*<24.39 >* | 1.37 t 0.24<br>**(38.92** ± **15.07)**<br>*<25.92>* | 1.35 ± 0.22<br>**(36.93** ± **13.02)**<br>*<27.08>* | 1.33 ± 0.17<br>**(31.75** ± **13.70)**<br>*<27.60>* |
| enbrel 9mg/kg | 1.06 ± 0.04 | 1.38 ± 0.20<br>**(36.48** ± **15.50)**<br>*<30.52 >* | 1.36 ± 0.22<br>**(35.30** ± **14.25)**<br>*<31.53 >* | 1.34 ± 0.21<br>**(33.88** ± 13.57)<br>*<35.50>* | 1.34 ± 0.20<br>**(32.16** ± **15.12)**<br>*<36.49>* | 1.31 ± 0.16<br>**(27.63 ±10.87)**<br>*<36.99>* |
| Tripterygium Wilfordii Hook 1.5mglkg | 1.03 ± 0.05 | 1.43 ± 0.24<br>**(46.48** ± **16.88)**<br>*<11.47>* | 1.35 ± 0.19<br>**(37.22** ± **16.50)**<br>*<27.82 >* | 1.36 ± 0.19<br>**(38.09** ± **14.10)**<br>*<27.49 >* | 1.36 ± 0.20<br>**(39.06** ± **13.00)**<br>*<22.87 >* | 1.37 ± 0.19<br>**(40.19** ± 11.80)<br>*<8.33>* |
| ***P<0.05, **P<0.01 compared with control group ( ) is swell rate** (%) < > is *inhibition rate (%)* | | | | | | |

3. Treatement effect of ginsenoside Compound-K on Collagen Type II (CII) induced arthritis in rats (not claimed)

3.1 Methods of experiments:

Collagen Type II (CII) and Freund's Adjuvant Incomplete were ground into emulsion and were injected subcutaneously into the back of the rats for immunization. Until the 19th day, most parts of the pedal joint of the rats began to swell. Picked 40 rats out which were with swelled pedal joint and divided them into 5 groupssubsequently, i.e. 8 rats in each group. They are respectively inflammation-induced control group: Ginsenoside Compound-K, 10mg/kg, iv, medium dose group: Ginsenoside Compound-K, 5mg/kg, iv, small dose group: Ginsenoside Compound-K, 2.5mg/kg, iv, Enbrel group: 9mg/kg, sc, Tripterygium Wilfordii Hook group: 1.5mg/kg, po. and blank control group: physiological saline 10mg/kg, iv. The rats were administered for 8 days consecutively. The swell value of the foot was measured respectively by using hydroplethismometer before inflammation induced and at different time after the onset of symptoms, then calculated swell rate and prohibition rate, and compared the differences among groups with t test.

3.2 Results

The results were shown in table 6. Ginsenoside Compound-K could be able to cure Collagen Type II induced arthritis. The strongest effect was observed in the large dose group, the best effect was achieved on day 31 with the highest inhibition rate 27.67%, which was higher than Tripterygium Wilfordii Hook group and amount to the Enbrel group.

**Table 6. Treatement Effect of Ginsenoside Compound-K on Collagen Type II (CII) Induced Arthritis**

| (%, **n=8,** x±s) | The swell vale at different days after inflammation-induced (ml) | | | | | | |
|---|---|---|---|---|---|---|---|
| | 0day | 20day | 22day | 23day | 25day | 28day | 31day |
| Blank control | 1.03 ±0.07 | 1.01 ± 0.12 | 1.01 ± 0.08 | 1.07 ± 0.10 | 1.06 ± 0.06 | 1.04 ± 0.09 | 1.04 ± 0.08 |

(continued)

| (%, **n=8,** x±s) | The swell vale at different days after inflammation-induced (ml) | | | | | | |
|---|---|---|---|---|---|---|---|
| | 0day | 20day | 22day | 23day | 25day | 28day | 31day |
| Inflammation-induceded control | 1.08 ±0.06 | 1.67 ± 0.09<br>**(54.03** ± **10.64)** | 1.73 ± 0.18<br>**(60.23** ± **20.06)** | 1.75 ± 0.14<br>**(62.26** ± **18.40)** | 1.75 ± 0.20<br>**(61.69** ± **18.90)** | 1.64 ± 0.17<br>**(51.64** ± **17.41)** | 1.61 ± 0.07<br>**(49.00** ± **8.66)** |
| Large dose 10mg/kg | 1.04 ± 0.19 | <1.52 ± 0.19<br>**(47.22** ± **18.60)**<br>*<12.61>* | 1.54 ± 0.12<br>**(50.43** ± **9.45)**<br>*<16.28>* | 1.54 ± 0.10<br>**(50.25** ± **22.06)**<br>*<19.28>* | 1.54 ± 0.12<br>**(50.62** ± **21.43)**<br>*<17.95>* | **1.44** ± 0.13<br>**(40.23** ± **16.26)**<br>*<22.09>* | 1.38 ± 0.11<br>**(35.44** ± **22.50)**<br>*<27.67>* |
| Medium dose 5mg/kg | 1.03 ± 0.05 | 1.52 ± 0.21<br>**(48.97** ± **27.82**<br>*<9.35>* | 1.56 ± 0.15<br>**(52.77** ± **21.55)**<br>*<12.39>* | 1.58 ± 0.13<br>**(53.62** ± **14.27)**<br>*<13.87>* | 1.56 ± 0.19<br>**(52.06 18.56)**<br>*<15.62>* | 1.49 ± 0.28<br>**(44.84** ± **25.91)**<br>*<13.17>* | 1.45 ± 0.13<br>**(41.34** ± **13.83)**<br>*<15.64>* |
| Low dose 2.5gmg/kg | 1.03 ± 0.05 | 1.48 ± 0.11<br>**(43.79** ± **12.08**<br>*<18.95>* | 1.53 ± 0.06<br>**(48.22** ± **10.27)**<br>*<19.94>* | 1.56 ± 0.06<br>**(51.23** ± **9.82)**<br>*<17.70>* | 1.56 ± 0.16<br>**(51.66** ± **18.36)**<br>*<16.26>* | 1.45 ± 0.10<br>**(41.04** ± **10.92)**<br>*<20.52>* | 1.42 ± 0.10<br>**(37.86** ± **11.18)**<br>*<22.73>* |
| enbrel 9mg/kg | 1.00 ± 0.08 | 1.51 ± 0.11<br>**(51.65** ± **15.35**<br>*<4.41>* | 1.48 ± 0.15<br>**(49.63** ± **25.60)**<br>*<17.61>* | 1.48 ± 0.12<br>**(49.20** ± **19.86)**<br>*<20.98>* | 1.46 ± 0.11<br>**(46.43** ± **15.37)**<br>*<24.74>* | 1.36 ± 0.09<br>**(37.24** ± 17.23)<br>*<27.66>* | 1.35 ± 0.08<br>**(36.24** ± **14.64)**<br>*<26.03>* |
| Tripterygium Wilfordii Hook 1.5mg/kg | 1.00 ± 0.12 | 1.50 ± 0.17<br>**(51.18** ± **18.93**<br>*<5.28>* | 1.52 ± 0.09<br>**(54.14** ± **23.10)**<br>*<10.12>* | 1.55 ± 0.12<br>**(56.57** ± **23.30**<br>*<9.13>* | 1.48 ± 0.11<br>**(49.69** ± **22.81**<br>*<19.45>* | 1.39 ± 0.12<br>**(41.18** ±**22.50**<br>*<20.25>* | 1.37 ± 0.12<br>**(38.71** ± **22.60)**<br>*<20.99>* |
| ***P<0.05, **P<0.01 compared with inflammationinduced control group ( ) is swell rate (%) < >** *is inhibition rate (%)* | | | | | | | |

## Claims

1. Use of ginsenoside Compound-K in manufacturing of medicaments for prevention of rheumatoid arthritis.

## Patentansprüche

1. Verwendung von Ginsenosid-Verbindung-K bei Herstellung von Medikamenten für die Prävention von rheumatoider Arthritis.

## Revendications

1. Utilisation de composé K de ginsénoside dans la fabrication de médicaments pour prévenir l’arthrite rhumatoïde.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 1570733 A
- WO 2004058796 A1
- KR 20060067900 A1